(19) Europäisches Patentamt
European Patent Office
Office européen des brevets

(11) EP 2 878 271 A1

(12) EUROPEAN PATENT APPLICATION
published in accordance with Art. 153(4) EPC

(43) Date of publication:
03.06.2015 Bulletin 2015/23

(51) Int Cl.:
A61B 8/12 (2006.01)

(21) Application number: 13823647.6

(22) Date of filing: 28.06.2013

(86) International application number:
PCT/JP2013/067911

(87) International publication number:
WO 2014/017255 (30.01.2014 Gazette 2014/05)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 27.07.2012 JP 2012167636

(71) Applicant: OLYMPUS MEDICAL SYSTEMS CORP.
Tokyo 151-0072 (JP)

(72) Inventor: NOGUCHI, Hiromasa
Tokyo 151-0072 (JP)

(74) Representative: Schicker, Silvia
Wuesthoff & Wuesthoff
Patentanwälte PartG mbB
Schweigerstraße 2
81541 München (DE)

(54) **ULTRASOUND OBSERVATION APPARATUS, METHOD FOR OPERATING ULTRASOUND OBSERVATION APPARATUS AND PROGRAM FOR OPERATING ULTRASOUND OBSERVATION APPARATUS**

(57) In order to accurately detect a position of an artificial material inserted into a body of subject, with a simple structure, and to improve visibility of the artificial material in an ultrasonic image, an ultrasonic observation apparatus includes a frequency analyzer that analyzes a frequency of the echo signal to calculate a frequency spectrum, a feature data calculator that calculates feature data reflecting a frequency spectrum shape specific to an artificial material having a spatially regular structure, and an artificial material-highlighted image data generator that generates, based on the feature data, an image in which the artificial material is highlighted.

FIG.3

## Description

Field

The present invention relates to an ultrasonic observation apparatus for observing tissue of a subject by using an ultrasonic wave, an operation method of the ultrasonic observation apparatus, and an operating program for the ultrasonic observation apparatus.

Background

An ultrasonic observation system that observes tissue of a subject by using ultrasonic waves sometime uses a puncture needle which is a treatment tool for making a puncture in a region of interest of the subject. When using the puncture needle, it is very important to make a puncture in the region of interest accurately while checking, in real time, a position of a needle tip of the puncture needle and thus, good visibility of the needle tip in an ultrasonic image is required.

Conventionally, a technology for accurately grasping the position of the needle tip of the puncture needle, a technique for intensifying an artificial material by performing correlation operation between a reception signal and a reference signal obtained by capturing an image of the artificial material is disclosed (see, for example, Patent Literature 1). Further, a technique for determining the position of the puncture needle using a position sensor is disclosed (see, for example, Patent Literature 2).

Citation List

Patent Literature

Patent Literature 1: Japanese Laid-open Patent Publication No. 2006-175006
Patent Literature 2: Japanese Laid-open Patent Publication No. 2005-323669

Summary

Technical Problem

In the technique disclosed in Patent Literature 1, however, it is difficult to calculate the correlation when the puncture needle is deviated from a specified area, and thus a user may lose the needle tip in the image.

In the technique disclosed in Patent Literature 2, providing the position sensor makes the system complicated and costly.

The present invention has been made in view of the foregoing and an object of the present invention is to provide an ultrasonic observation apparatus capable of accurately detecting the position of an artificial material inserted into the body of the subject, with a simple structure, and having a display parameter capable of improving visibility of the artificial material in an ultrasonic image, an operation method of the ultrasonic observation apparatus, and an operating program for the ultrasonic observation apparatus.

Solution to Problem

To solve the problem described above and achieve the object, an ultrasonic observation apparatus according to the invention receives an echo signal of an ultrasonic wave from an ultrasonic probe for transmitting an ultrasonic wave to a subject and receiving the ultrasonic wave reflected from the subject and performs signal processing on the echo signal. The ultrasonic observation apparatus includes: a frequency analyzer that analyzes a frequency of the echo signal to calculate a frequency spectrum; a feature data calculator that calculates feature data reflecting a frequency spectrum shape specific to an artificial material having a spatially regular structure; and an artificial material-highlighted image data generator that generates, based on the feature data, an image in which the artificial material is highlighted.

In the above invention, the ultrasonic observation apparatus according the invention further includes an artificial material detector that detects, based on the feature data calculated by the feature data calculator, information on the artificial material. The artificial material-highlighted image data generator generates, based on the feature data and a detection result by the artificial material detector, the image in which the artificial material is highlighted.

According to the ultrasonic observation apparatus of the invention, in the above invention, the artificial material is a puncture needle on a surface of a distal end of which a plurality of concave portions are arranged to form a spatially regular pattern.

According to the ultrasonic observation apparatus of the invention, in the above invention, the ultrasonic probe can receive an ultrasonic wave satisfying

$2d\sin\theta = n\lambda$ (n is an integer)

where an interval between the concave portions adjacently disposed is d, a wavelength of the ultrasonic wave is $\lambda$, and, an incident angle of the ultrasonic wave incident on a surface of the puncture needle is $\theta$.

According to the ultrasonic observation apparatus of the invention, in the above invention, the feature data calculator calculates, as the feature data, a frequency band that imparts an intensity within a specified range from a maximum value of the intensity of the frequency spectrum.

According to the ultrasonic observation apparatus of the invention, in the above invention, the feature data calculator approximates a frequency band in the frequency spectrum that includes a frequency characterizing the artificial material, by a quadratic curve through regression analysis, and calculates, as the feature data, a coefficient of a squared term in the quadratic curve.

According to the ultrasonic observation apparatus of the invention, in the above invention, the feature data calculator approximates a frequency band in the frequency spectrum that includes a frequency characterizing the artificial material, by a straight line through regression analysis, and calculates, as the feature data, a slope of the straight line.

According to the ultrasonic observation apparatus of the invention, in the above invention, the feature data calculator approximates a frequency band in the frequency spectrum that includes a frequency characterizing the artificial material, by a straight line through regression analysis, and calculates, as the feature data, an intercept of the straight line.

In the above invention, the ultrasonic observation apparatus according to the invention further includes a feature data information storage unit that stores feature data information which is information required for the feature data calculator to calculate the feature data. The feature data calculator calculates, based on information stored in the feature data information storage unit, the feature data.

An operation method according to the invention is an operation method of an ultrasonic observation apparatus that receives an echo signal from an ultrasonic probe for transmitting an ultrasonic wave to a subject and receiving the ultrasonic wave reflected from the subject and performs signal processing on the echo signal. The operation method includes: analyzing, by a frequency analyzer, a frequency of the echo signal to calculate a frequency spectrum; calculating, by a feature data calculator, feature data reflecting a frequency spectrum shape specific to an artificial material having a spatially regular structure; and generating, by an artificial material-highlighted image data generator, an image in which the artificial material is highlighted, based on the feature data.

An operating program according to the invention is an operating program for an ultrasonic observation apparatus that receives an echo signal from an ultrasonic probe for transmitting an ultrasonic wave to a subject and receiving the ultrasonic wave reflected from the subject and performs signal processing on the echo signal. The program causes the ultrasonic observation apparatus to perform: analyzing, by a frequency analyzer, a frequency of the echo signal to calculate a frequency spectrum; calculating, by a feature data calculator, feature data reflecting a frequency spectrum shape specific to an artificial material having a spatially regular structure; and generating, by an artificial material-highlighted image data generator, an image in which the artificial material is highlighted, based on the feature data.

Advantageous Effects of Invention

According to the invention, feature data reflecting a frequency spectrum shape specific to an artificial material having a specially regular structure is calculated, and then an image in which the artificial material is highlighted is generated based on the calculated feature data. With this configuration, it is possible to provide an ultrasonic observation apparatus capable of accurately detecting the position of an artificial material inserted into the body of the subject, with a simple structure, and having a display parameter capable of improving visibility of the artificial material in an ultrasonic image, an operation method of the ultrasonic observation apparatus, and an operating program for the ultrasonic observation apparatus.

Brief Description of Drawings

FIG. 1 is an exemplary view illustrating a configuration of an ultrasonic observation system including an ultrasonic observation apparatus according to an embodiment of the present invention;

FIG. 2 is an exemplary view illustrating a configuration of a distal end of an insertion unit of an ultrasonic endoscope;

FIG. 3 is a block diagram illustrating a configuration of the ultrasonic observation apparatus according to the embodiment of the present invention;

FIG. 4 is a view illustrating a relationship between a reception depth and an amplification factor of an echo signal;

FIG. 5 is a view illustrating a relationship between the reception depth and amplification factor in amplification processing performed by an amplification corrector that the ultrasonic observation apparatus according to the embodiment of the present invention includes;

FIG. 6 is a view illustrating an example of a frequency spectrum calculated by a frequency analyzer that the ultrasonic observation apparatus according to the embodiment of the present invention includes;

FIG. 7 is an exemplary view illustrating a brief overview of attenuation correction processing performed by an attenuation corrector that the ultrasonic observation apparatus according to the embodiment of the present invention includes;

FIG. 8 is a partially enlarged view illustrating a distal end of a puncture needle;

FIG 9 is an exemplary view illustrating a state where a scattered light at the distal end of the puncture needle is enhanced;

FIG. 10 is a view illustrating a frequency spectrum (first example) of an echo signal at a point-like region in a B-mode image;

FIG. 11 is a view illustrating a frequency spectrum (second example) of an echo signal at a point-like region in a B-mode image;

FIG. 12 is a view explaining a brief overview (first example) of feature data calculation processing and artificial material detection processing in the embodiment of the present invention;

FIG. 13 is a view explaining a brief overview (second example) of feature data calculation processing and artificial material detection processing in the embodiment of the present invention;

FIG. 14 is a flowchart illustrating a brief overview of processing to be performed in the ultrasonic endoscope system;

FIG. 15 is a flowchart illustrating a brief overview of processing to be performed by the frequency analyzer;

FIG. 16 is an exemplary view illustrating data arrangement of one acoustic ray;

FIG. 17 is a view illustrating a display example of an artificial material-highlighted image displayed on a display unit;

FIG. 18 is a view explaining a brief overview (first example) of the feature data calculation processing and artificial material detection processing in a first modification of the embodiment of the present invention;

FIG. 19 is a view explaining a brief overview (second example) of the feature data calculation processing and artificial material detection processing in the first modification of the embodiment of the present invention;

FIG. 20 is a view explaining a brief overview (first example) of the feature data calculation processing and artificial material detection processing in a second modification of the embodiment of the present invention;

FIG. 21 is a view explaining a brief overview (second example) of the feature data calculation processing and artificial material detection processing in the second modification of the embodiment of the present invention;

FIG. 22 is a view explaining a brief overview (first example) of the feature data calculation processing and artificial material detection processing in a third modification of the embodiment of the present invention;

FIG. 23 is a view explaining a brief overview (second example) of the feature data calculation processing and artificial material detection processing in the third modification of the embodiment of the present invention;

FIG. 24 is a view illustrating feature data information (first example) stored in a feature data information storage unit of the ultrasonic observation apparatus according to a fourth modification of the embodiment of the present invention; and

FIG. 25 is a view illustrating feature data information (second example) stored in the feature data information storage unit of the ultrasonic observation apparatus according to the fourth modification of the embodiment of the present invention.

Description of Embodiments

Modes for carrying out the present invention (hereinafter referred to as "embodiments") will be described below with reference to the accompanying drawings.

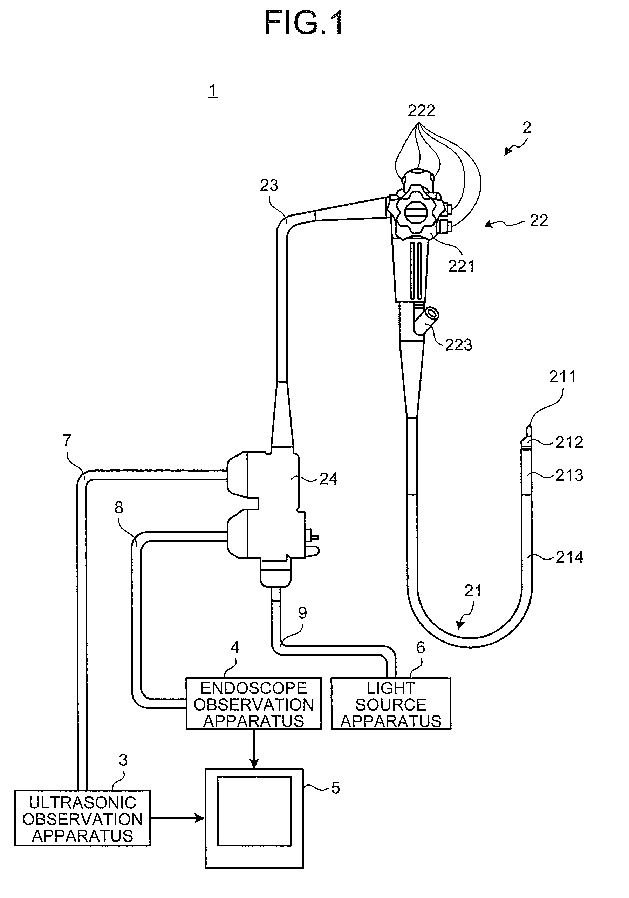

FIG. 1 is an exemplary view illustrating a configuration of an ultrasonic endoscope system as an example of an ultrasonic observation system including an ultrasonic observation apparatus according to the embodiment of the present invention. An ultrasonic endoscope system 1 of FIG. 1 includes an ultrasonic endoscope 2 capable of being inserted into a subject and provided with functions of transmitting a pulse-like ultrasonic wave and receiving an externally reflected ultrasonic wave (echo) as well as a function of capturing the subject to generate an image signal, an ultrasonic observation apparatus 3 configured to perform specified processing to an electrical echo signal received from the ultrasonic endoscope 2 to generate an ultrasonic image, an endoscope observation apparatus 4 configured to generate an endoscope image based on the image signal generated by the ultrasonic endoscope 2, a display apparatus 5 realized by using a display panel formed of liquid crystal or organic EL and capable of displaying the image generated by the ultrasonic observation apparatus 3 and the endoscope observation apparatus 4, a light source apparatus 6 configured to supply illumination light to be emitted outside from a distal end of the ultrasonic endoscope 2, an ultrasonic cable 7 configured to electrically connect the ultrasonic endoscope 2 and ultrasonic observation apparatus 3, a video cable 8 configured to electrically connect the ultrasonic endoscope 2 and the endoscope observation apparatus 4, and an optical fiber cable 9 provided with an optical fiber that connects the ultrasonic endoscope 2 and the light source apparatus 6 to supply the illumination light generated by the light source apparatus 6 to the ultrasonic endoscope 2.

The ultrasonic endoscope 2 includes an insertion unit 21 to be inserted into a body, an operation unit 22 coupled to a base end side of the insertion unit 21, a universal cable 23 extending from the operation unit 22, and a connector 24 provided at a distal end of the universal cable 23.

The insertion unit 21 includes an ultrasonic probe 211 provided at a distal end thereof, a hard portion 212 formed of a hard member and connected to the ultrasonic probe 211, a bending portion 213 bendably connected to a base end side of the hard portion 212, and a flexible tube portion 214 having flexibility and provided at a base end side of the bending portion 213. Although not illustrated, there are provided, inside the insertion unit 21, a light guide that transmits the illumination guide, a plurality of signal lines that transmits various electrical signals, and a treatment tool passage for insertion of a treatment tool.

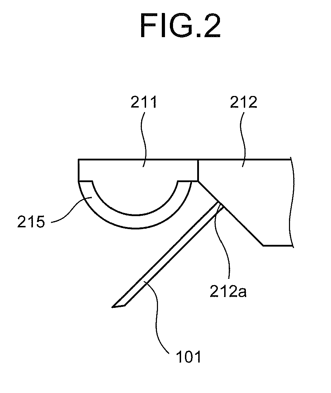

FIG. 2 is an exemplary view illustrating a configuration of the distal end of the insertion unit 21. The ultrasonic probe 211 is a convex type ultrasonic probe and has a transducer unit 215 in which a plurality of ultrasonic transducers are regularly arranged. The ultrasonic transducer includes an acoustic lens, a piezoelectric element, and a matching layer and is configured to acquire an ultrasonic signal contributing more to an ultrasonic tomographic image of an inside of a subject's body than to a body wall of the subject. The plurality of ultrasonic transducers are arranged so as to form a convex circular arc.

A treatment tool channel 212a allowing various treatment tool to extend is formed at a distal end of the hard portion 212. FIG. 2 illustrates an example in which a puncture needle 101 which is a treatment tool extends from the treatment tool channel 212a. An extending angle of the puncture needle 101 from the treatment tool channel 212a is defined depending on a type of the insertion unit 21. A mechanism for adjusting the extending angle of the puncture needle 101 can be provided in the insertion unit 21.

The ultrasonic probe 211 has functions of converting an electric pulse signal received from the ultrasonic observation apparatus 3 into an ultrasonic pulse (acoustic pulse signal) and converting an ultrasonic echo externally reflected by the subject into an electrical echo signal. The echo signal obtained as a result of the conversion performed by the ultrasonic probe 211 is transmitted to the ultrasonic observation apparatus 3 through the ultrasonic cable 7. A method of scanning the ultrasonic transducer employed by the ultrasonic probe 211 may be an electronic scanning method or a mechanical scanning method.

An imaging element (not illustrated) that captures an object positioned in a specified visual field to generate an image signal for an endoscope image is provided at the distal end of the hard portion 212. The image signal generated by the imaging element is transmitted to the endoscope observation apparatus 4 through the video cable 8.

The illumination light supplied from the light source apparatus 6 can be emitted from the distal end of the hard portion 212. Specifically, an illumination window (not illustrated) for emitting, therethrough, the illumination light transmitted from the light source apparatus 6 is provided at the distal end of the hard portion 212.

The operation unit 22 includes a bending knob 221 for operating the bending portion 213 in a bending manner in a vertical (up-down) or horizontal (left-right) direction, a plurality of operation members 222 for performing various operations, and a treatment tool insertion port 223 configured to communicate with the treatment tool passage formed in the insertion unit 21 so as to allow insertion of the treatment tool into the treatment tool passage.

Through the universal cable 23, a plurality of signal lines for transmitting an electric signal, an optical fiber for transmitting the illumination light, and the like are inserted, although not illustrated.

The connector 24 is configured to transmit and receive a signal to and from the ultrasonic observation apparatus 3, the endoscope observation apparatus 4, and the light source apparatus 6 through the ultrasonic cable 7, the video cable 8, and the optical fiber cable 9, respectively.

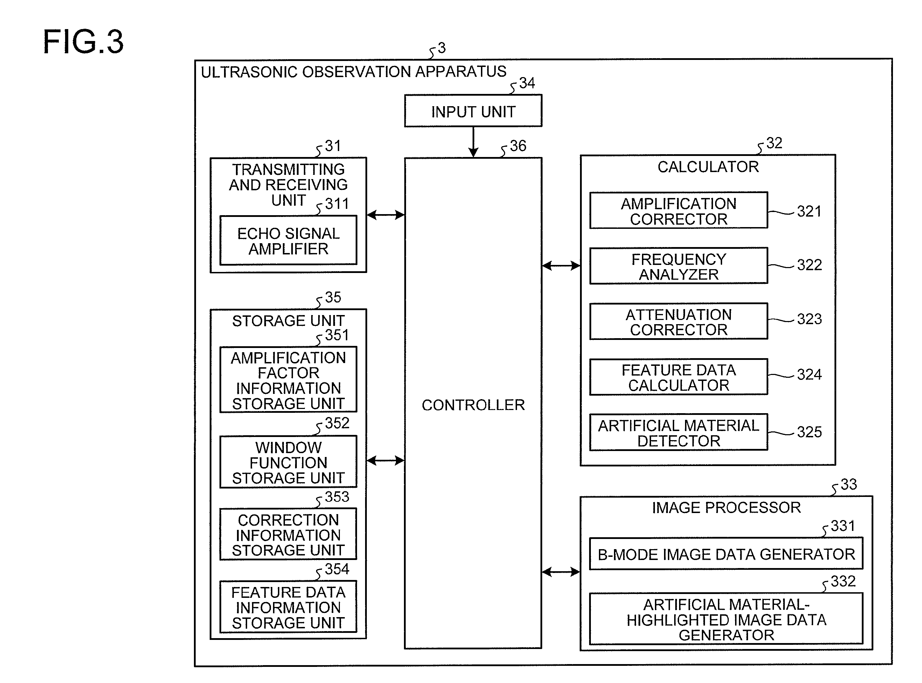

Next, reference will be made to a configuration of the ultrasonic observation apparatus 3. FIG. 3 is a block diagram illustrating a configuration of the ultrasonic observation apparatus 3. The ultrasonic observation apparatus 3 is an apparatus that receives an echo signal from the ultrasonic probe 211 and performs signal processing on the echo signal. The ultrasonic observation apparatus 3 includes a transmitting and receiving unit 31 configured to transmit and receive an electrical signal to and from the ultrasonic endoscope 2 and the display apparatus 5, a calculator 32 configured to perform specified calculation for the echo signal received from the ultrasonic endoscope 2, an image processor 33 configured to generate image data corresponding to the echo signal, an input unit 34 realized by using interfaces such as a keyboard, a mouse, and a touch panel and configured to receive various information as an input, a storage unit 35 for storing various information for observing the subject based on the ultrasonic wave, and a controller 36 configured to perform operation control of the ultrasonic observation apparatus 3.

The transmitting and receiving unit 31 is electrically connected to the ultrasonic probe 211 and configured to transmit a pulse signal to the ultrasonic probe 211 and receive an echo signal therefrom. Specifically, the transmitting and receiving unit 31 generates a pulse signal based on a previously set waveform and transmission timing and transmits the generated pulse signal to the ultrasonic probe 211.

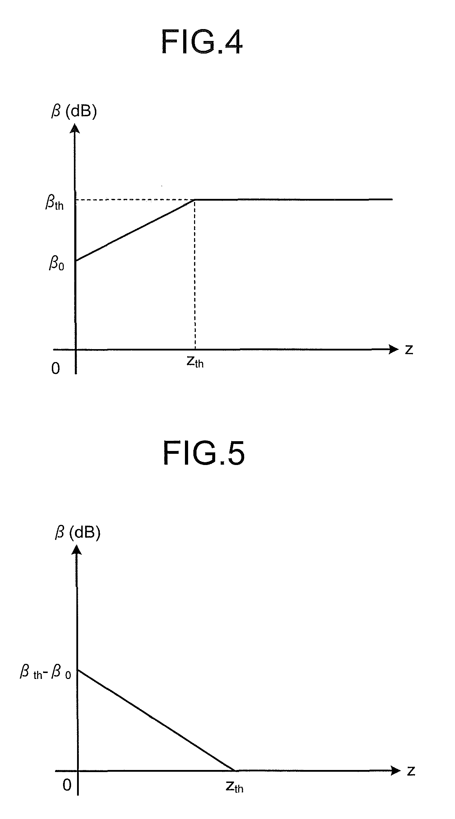

The transmitting and receiving unit 31 has an echo signal amplifier 311 that amplifies the echo signal. Specifically, the echo signal amplifier 311 performs STC (Sensitivity Time Control) correction in which an echo signal with a larger reception depth is amplified with a higher amplification factor. FIG. 4 is a view illustrating a relationship between the reception depth and amplification factor of the echo signal. A reception depth z illustrated in FIG. 4 is an amount calculated based on a time elapsed from a reception start time point of an ultrasonic wave. As illustrated in FIG. 4, an amplification factor $\beta$ (dB) is linearly increased from $\beta_0$ to $\beta_{th}$ ($> \beta_0$) with an increase in the reception depth z when the reception depth z is smaller than a threshold $z_{th}$. When the reception depth z is equal to or larger than a threshold $z_{th}$, the amplification factor $\beta$ assumes a certain value $\beta_{th}$. A value of the threshold $z_{th}$ is a value at which the ultrasonic signal received from the subject is almost attenuated and noise is thus dominant. More generally, the amplification factor $\beta$ may monotonically increase with an increase in the reception depth z when the reception depth z is smaller than the threshold $z_{th}$.

The transmitting and receiving unit 31 performs processing, such as filtering, on the echo signal amplified by the echo signal amplifier 311 and then A/D converts the resultant signal to generate and output a digital RF signal. In a case where the ultrasonic probe 211 electronically scans the plurality of ultrasonic transducers, the transmitting and receiving unit 31 has a multichannel circuit for beam synthesis corresponding to the plurality of ultrasonic transducers.

The calculator 32 includes an amplification corrector 321 configured to perform amplification correction to make the amplification factor constant with respect to the digital RF signal output from the transmitting and receiving unit 31 regardless of the reception depth, a frequency analyzer 322 configured to perform fast Fourier transform (FFT) to the digital RF signal that has been subjected to the amplification correction to analyze the frequency to thereby calculate a frequency spectrum, an attenuation corrector 323 configured to perform attenuation correction processing to the frequency spectrum calculated by the frequency analyzer 322 to reduce contribution of attenuation of the ultrasonic wave depending on the reception depth and frequency of the ultrasonic wave, a feature data calculator 324 configured to calculate feature data reflecting a frequency spectrum shape specific to an artificial material having a spatially regular structure as feature data reflecting the frequency spectrum that has been subjected to the attenuation correction by the attenuation corrector 323, and an artificial material detector 325 configured to detect, based on the feature data calculated by the feature data calculator 324, a reflection signal from the artificial material having a spatially regular structure as information of the artificial material.

FIG. 5 is a view illustrating a relationship between the reception depth and amplification factor in the amplification processing performed by the amplification corrector 321. As illustrated in FIG. 5, the amplification factor of the amplification processing performed by the amplification corrector 321 assumes a maximum value $\beta_{th} - \beta_0$ at a reception depth z of zero, is linearly reduced at a reception depth z of up to a threshold $z_{th}$, and is zero when the reception depth is equal to or larger than a threshold $z_{th}$. When the amplification corrector 321 performs amplification correction for a digital RF signal with such an amplification factor, the influence of the STC correction in the echo signal amplifier 311 can be offset, and a signal of the certain amplification factor $\beta_{th}$ can be output. Of course, the relationship between the reception depth z and the amplification factor $\beta$ achieved in the amplification corrector 321 varies in accordance with the relationship between the reception depth and amplification factor in the echo signal amplifier 311.

For each acoustic ray (line data), the frequency analyzer 322 applies fast Fourier transform to an FFT data group formed of a specified data amount to calculate a frequency spectrum at a plurality of positions (data positions) on an acoustic ray. The frequency spectrum tends to vary depending on tissue characteristics of a subject. The reason for this is that the frequency spectrum is correlated with a size, a density, an acoustic impedance, and the like of a subject as a scatterer scattering an ultrasonic wave. In the present embodiment, the "tissue characteristic" indicates, any of cancer, endocrine tumor, mucinous tumor, normal tissue, vascular channel, and the like.

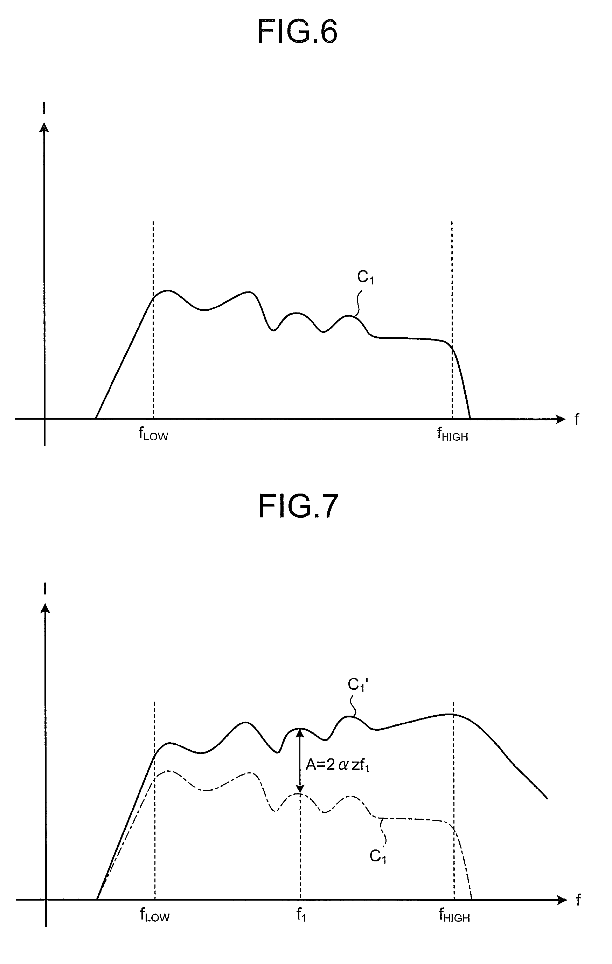

FIG. 6 is a view illustrating an example of the frequency spectrum calculated by the frequency analyzer 322. In FIG. 6, a horizontal axis f represents a frequency, and a vertical axis I represents an intensity. Here, the "intensity" is any of parameters such as voltage, electric power, sound pressure, acoustic energy, and the like. In a frequency spectrum curve $C_1$ illustrated in FIG. 6, a lower limit frequency $f_{LOW}$ and an upper limit frequency $f_{HIGH}$ of the frequency spectrum are parameters determined based on a frequency band of the ultrasonic probe 211, a frequency band of the pulse signal transmitted from the transmitting and receiving unit 31, and the like. For example, $f_{LOW}$ is set to 3 MHz, and $f_{HICH}$ is set to 10 MHz. In the present embodiment, the curve and the straight lines are formed of a set of discrete points.

FIG. 7 is an exemplary view illustrating a brief overview of the attenuation correction processing performed by the attenuation corrector 323. As illustrated in FIG. 7, the attenuation corrector 323 corrects the frequency spectrum curve $C_1$ by adding an attenuation amount A defined by the following expression (1) to an intensity I at all of frequencies f ($f_{LOw} < f < f_{HIGH}$) within a frequency band.

$$A = 2\alpha z f \qquad\qquad (1)$$

where $\alpha$ is an attenuation rate, z is the reception depth of an ultrasonic wave, and f is a frequency. The attenuation rate

$\alpha$ is determined according to a type of an object to be observed. For example, when the object to be observed is a living body, the attenuation rate $\alpha$ is in a range of from 0.0 to 1.0 (dB/cm/MHz) and, preferably, in a range of from 0.3 to 0.7 (dB/cm/MHz). When the object to be observed is a pancreas, $\alpha = 0.6$ (dB/cm/MHz) is determined. In the present embodiment, a configuration can also be employed in which the value of the attenuation rate $\alpha$ can be changed by an input from the input unit 34.

The application of the attenuation correction by the attenuation corrector 323 allows a new frequency spectrum curve $C_1'$ in which the contribution of attenuation caused due to propagation of the ultrasonic wave is reduced to be obtained. As a result, it is possible to prevent a problem in which the image becomes dark which results from a reduction in the signal intensity due to the influence of the attenuation on a region having the large reception depth and to obtain an image having uniform brightness over a screen.

The feature data calculator 324 calculates, as feature data, a bandwidth of a frequency corresponding to a specified range (hereinafter, referred to as intensity width) in which a peak intensity of the frequency spectrum that has been subjected to the attenuation correction by the attenuation corrector 323 is set as an upper limit value.

The artificial material detector 325 detects, based on the feature data calculated by the feature data calculator 324, a reflection signal from the artificial material having a spatially regular structure as information of the artificial material. As the artificial material having a spatially regular structure, there can be exemplified the puncture needle 101 and an ultrasonic marker which are to be described later.

The image processor 33 includes a B-mode image data generator 331 configured to generate B-mode image data from the echo signal and an artificial material-highlighted image data generator 332 configured to generate artificial material-highlighted image data in which a position of the artificial material is highlighted when the artificial material detector 325 detects a reflection signal from the artificial material. The echo signal received from the ultrasonic endoscope 2 is data based on polar coordinates. Thus, the image processor 33 converts the polar coordinates into orthogonal coordinates so as to generate image data to be added to each pixel.

The B-mode image data generator 331 performs, for the digital signal, signal processing using a known technique such as a bandpass filter, a logarithmic conversion, gain processing or contrast processing and generates the B-mode image data by decimating the data according to a data step width determined by a display range of the image on the display apparatus 5. The B-mode image is a grayscale image in which values of R (red), G (green), and B (blue), which are variables when an RGB color system is employed as a color space, are matched to one another. A region of interest in the B-mode image can be arbitrarily set by a user through the input unit 34.

The artificial material-highlighted image data generator 332 generates the artificial material-highlighted image data by adding, to the artificial material, a color easily distinguished from its surrounding portion or by increasing brightness of the artificial material as compared to the surrounding portion.

The input unit 34 is realized by using interfaces such as a keyboard, a mouse, and a touch panel. The input unit 34 is configured to receive an input of information designating a region of interest from a user of the ultrasonic observation apparatus 3 who views the image generated by the image processor 33.

The storage unit 35 includes an amplification factor information storage unit 351 for storing information of the amplification factor to be referred to when the echo signal amplifier 311 and the amplification corrector 321 perform the amplification processing, a window function storage unit 352 for storing a window function to be used in the frequency analysis processing performed by the frequency analyzer 322, a correction information storage unit 353 for storing correction information (including the above equation (1)) to be referred to when the attenuation corrector 323 performs processing, and a feature data information storage unit 354 for storing information on the feature data to be referred to when the artificial material detector 325 detects the artificial material.

The amplification factor information storage unit 351 stores the relationships between the amplification factor and reception depth, which are illustrated in FIGS. 4 and 5. The window function storage unit 352 stores at least one of the window functions such as Hamming, Hanning, and Blackman.

The storage unit 35 is realized using a ROM storing an operating program of the ultrasonic observation apparatus 3, a program for operating a specified OS, and the like in advance, a RAM storing calculation parameters and data for each processing, or the like.

The controller 36 is realized using a CPU having a calculation function and a control function. The controller 36 reads out the information stored in the storage unit 35 and various programs including the operating program of the ultrasonic observation apparatus 3 from the storage unit 35 to execute various calculation processing related to a method of operating the ultrasonic observation apparatus 3 to thereby perform overall control for the ultrasonic observation apparatus 3.

The operating program of the ultrasonic observation apparatus 3 can also be stored on a computer readable recording medium such as a hard disk, a flash memory, a CD-ROM, a DVD-ROM, or a flexible disk to be widely distributed.

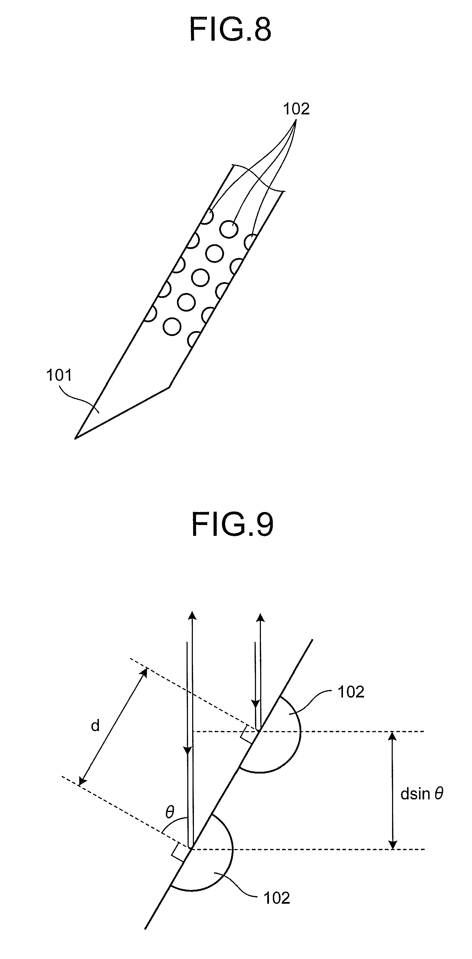

Next, reference will be made to a configuration of a distal end of the puncture needle 101 which is a treatment tool applied to the ultrasonic endoscope system 1. FIG. 8 is a partially enlarged view illustrating the distal end of the puncture needle 101. A plurality of minute concave portions 102 each having substantially a concave spherical surface are arranged in the distal end of the puncture needle 101 to form a spatially regular pattern. The plurality of concave portions 102 are provided for the purpose of effectively scattering the ultrasonic wave transmitted from the ultrasonic probe 211 so as to easily visualize the ultrasonic wave in the B-mode image. In the example of FIG. 8, the plurality of concave portions 102 are arranged in such a manner that they are equally spaced apart from the adjacent ones.

When the ultrasonic wave is transmitted to the puncture needle 101, each of the concave portions 102 scatters an acoustic wave to function as a point sound source. Assuming that a wavelength of the ultrasonic wave (transmission wave) transmitted from the ultrasonic probe 211 is $\lambda$, an interval between adjacent concave portions 102 is d, and an incident angle (angle between an incident direction of the transmission wave and a normal direction of a surface of the puncture needle 101) of the transmission wave incident on a surface of the puncture needle 101 is $\theta$, only a wavelength of the scattered wave at the distal end of the puncture needle 101 that satisfies the following conditional expression (2) is enhanced (see FIG. 9).

$$2d\sin\theta = n\lambda \qquad\qquad\qquad (2)$$

where n of the right-hand side is an integer. The ultrasonic probe 211 can effectively receive the scattered wave that satisfies the expression (2).

In general, the transmission wave includes waves of various frequencies; however, the number of integers n that satisfy the expression (2) is very few. Therefore, the ultrasonic wave scattered at a portion having the spatially regular structure like the distal end of the puncture needle 101, has a narrow band frequency spectrum. Thus, the ultrasonic observation apparatus 3 can distinguish an echo signal from the artificial material like the distal end of the puncture needle 101 from an echo signal from a living tissue by performing signal processing using the frequency spectrum.

Although the artificial material having a spatially regular structure is assumed to be the puncture needle 101 in the following descriptions, it goes without saying that this is merely one example.

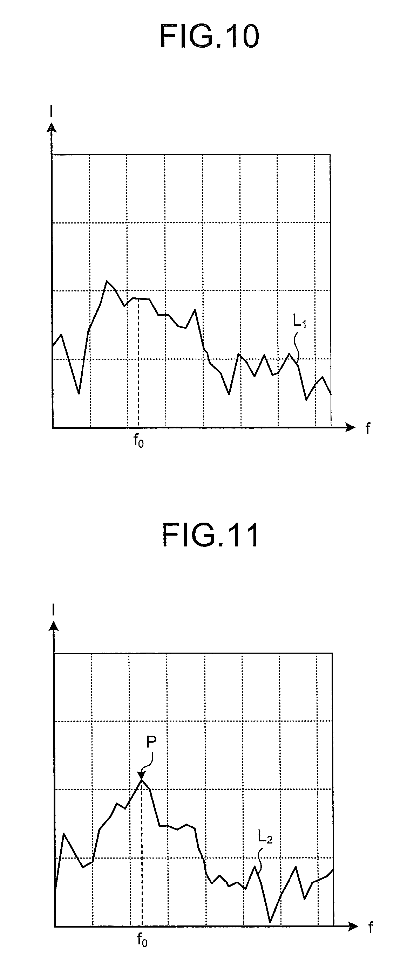

Next, reference will be made to a feature of processing to be performed by the ultrasonic observation apparatus 3. FIGS. 10 and 11 are views each illustrating a frequency spectrum of an echo signal at a point-like region in the B-mode image. Specifically, FIG. 10 illustrates a frequency spectrum when the point-like region corresponds to a living tissue, and FIG. 11 illustrates a frequency spectrum when the point-like region corresponds to the distal end of the puncture needle 101. The two frequency spectrums are acquired at the same depth and have the same level of influence such as attenuation due to propagation or energy migration to a harmonic sound. Thus, it can be said that a difference in the frequency spectrum is mainly caused by a difference in characteristics of a reflector. The "point-like region" mentioned here means an extremely minute region having a certain degree of breadth including the surrounding area. Thus, even when the point-like region corresponds to a region where the distal end of the puncture needle 101 is positioned, it may include small amount of information of the living tissue around the distal end of the puncture needle 101.

As can be seen from a comparison between a spectrum curve $L_1$ of FIG. 10 and a spectrum curve $L_2$ of FIG. 11, the spectrum curve $L_2$ assumes a shape having a peak at a position P corresponding to a frequency $f_0$, while the spectrum curve $L_1$ assumes a downward sloping linear shape around the frequency $f_0$. It can be considered that the shape of the spectrum curve $L_2$ occurs in a reflection signal from a portion forming the spatially regular pattern and results from enhancement of the scattered wave caused due to existence of the plurality of concave portions 102 of the puncture needle 101. On the other hand, the spectrum curve $L_1$ does not have a peak in as a narrow band as in the spectrum curve $L_2$. This is because the living tissue is not spatially regularly structured as much as the distal end of the puncture needle 101.

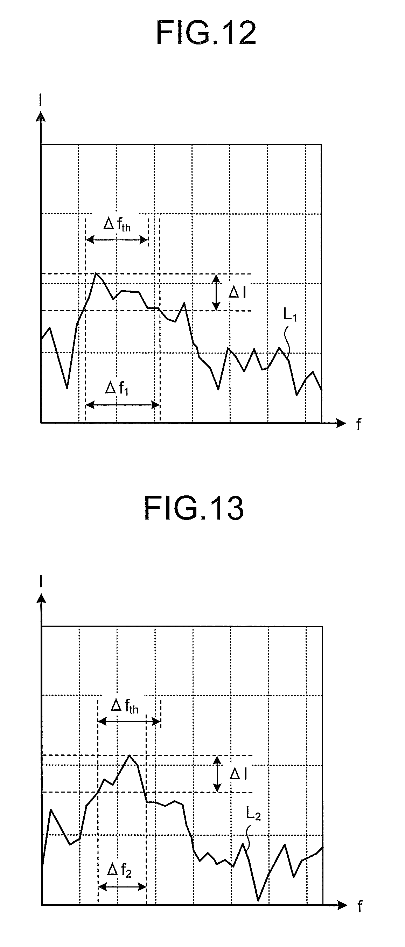

FIGS. 12 and 13 are views each explaining a brief overview of the feature data calculation processing and artificial material detection processing in the present embodiment. The feature data calculator 324 calculates, as feature data, a bandwidth $\Delta f$ of a frequency f corresponding to an intensity width $\Delta I$ in which a peak value of the frequency spectrum is set as an upper limit. The artificial material detector 325 compares the bandwidth $\Delta f$ calculated as the feature data by the feature data calculator 324 with a threshold value $\Delta f_{th}$ to determine whether or not the frequency spectrum is a frequency spectrum of the distal end of the puncture needle 101. Specifically, when the bandwidth $\Delta f$ is smaller than $\Delta f_{th}$, the artificial material detector 325 determines that the frequency spectrum is a frequency spectrum of the distal end of the puncture needle 101.

A bandwidth $\Delta f_1$ of the spectrum curve $L_1$ illustrated in FIG. 12 is larger than $\Delta f_{th}$ ($\Delta f_1 > \Delta f_{th}$). On the other hand, a bandwidth $\Delta f_2$ of the spectrum curve $L_2$ illustrated in FIG. 13 is smaller than $\Delta f_{th}$ ($\Delta f_2 < \Delta f_{th}$).

In this case, the artificial material detector 325 detects that a substance corresponding to the spectrum curve $L_2$ is the distal end of the puncture needle 101.

As described above, by using the frequency spectrum, the ultrasonic observation apparatus 3 can accurately detect the artificial material having a spatially regular structure like the distal end of the puncture needle 101 in the B-

EP 2 878 271 A1

mode image.

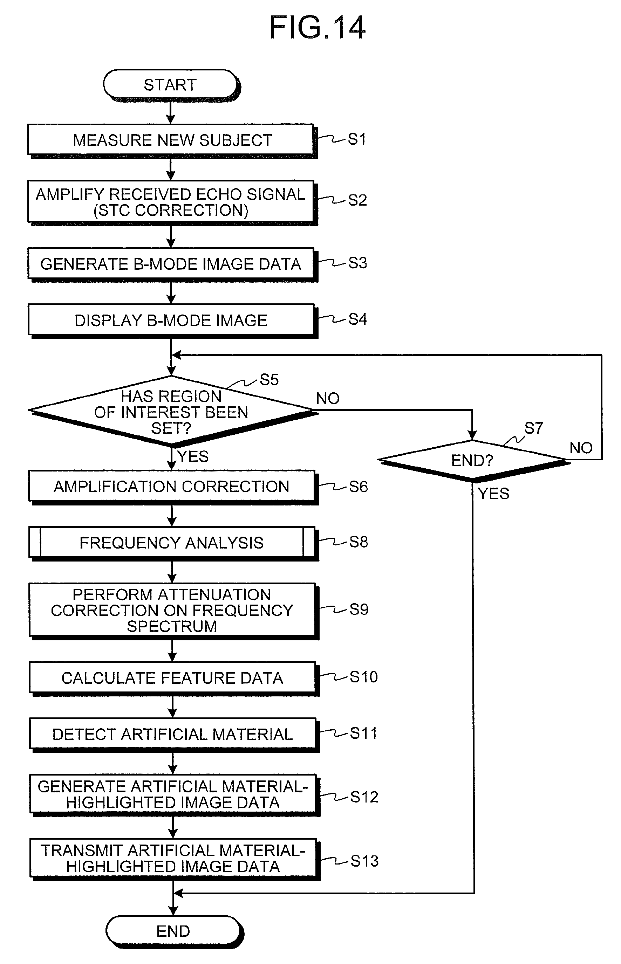

FIG. 14 is a flowchart illustrating a brief overview of processing to be performed in the ultrasonic endoscope system 1 having the configuration described above. First, the ultrasonic endoscope 2 measures a new subject using the ultrasonic probe 211 (step S1).

Subsequently, the echo signal amplifier 311 receiving an echo signal from the ultrasonic probe 211 amplifies the echo signal (step S2). Here, the echo signal amplifier 311 performs the amplification based on the relationship between the amplification factor $\beta$ and reception depth z illustrated in FIG. 4.

Then, the B-mode image data generator 331 generates B-mode image data using an echo signal for a B-mode image output from the transmitting and receiving unit 31 (step S3).

Next, the controller 36 controls the display apparatus 5 to display a B-mode image corresponding to the B-mode image data generated by the B-mode image data generator 331 (step S4).

Thereafter, when a region of interest is set through the input unit 34 (Yes in step S5), the amplification corrector 321 performs correction to make the amplification factor constant with respect to the signal output from the transmitting and receiving unit 31 regardless of the reception depth (step S6). Here, the amplification corrector 321 performs amplification processing based on the relationship between the amplification factor $\beta$ and reception depth z illustrated in FIG. 5. The region of interest may be set as a region corresponding to the entire B-mode image.

On the other hand, when no area of interest is set (No in step S5), the ultrasonic observation apparatus 3 terminates the processing when an instruction for terminating the processing is input through the input unit 34 (Yes in step S7). On the contrary, when no area of interest is set (No in step S5), the ultrasonic observation apparatus 3 returns to step S5 when an instruction for terminating the processing is not input through the input unit 34 (No in step S7).

After step S6, the frequency analyzer 322 calculates a frequency spectrum by analyzing the frequency through FFT calculation (step S8). In step S8, the entire image area can also be set as a region of interest.

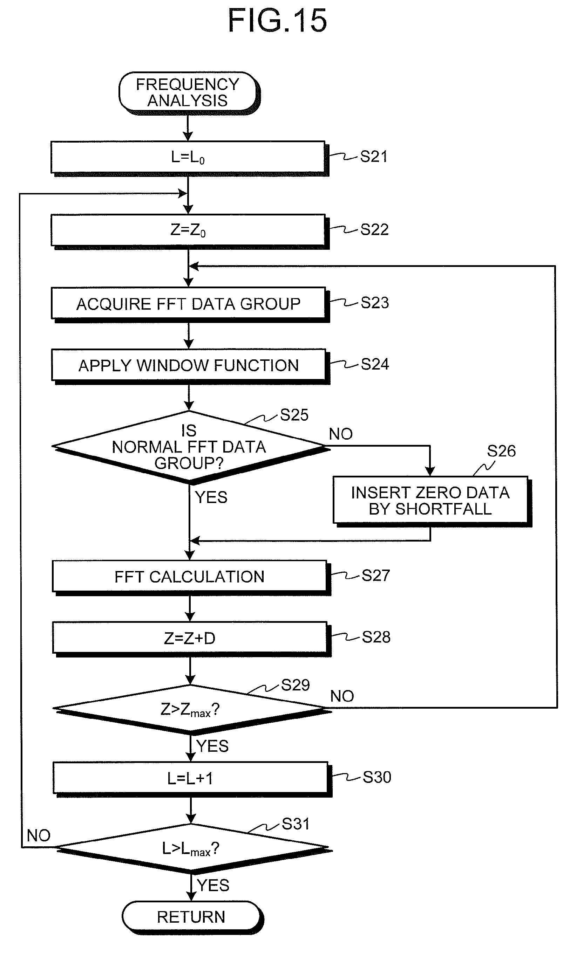

Here, the processing performed by the frequency analyzer 322 (step S8) will be described in detail with reference to a flowchart illustrated in FIG. 15. First, the frequency analyzer 322 sets an acoustic ray number L of an acoustic ray which is a first analysis target as an initial value $L_0$ (step S21). The initial value $L_0$ may be imparted to, for example, an acoustic ray initially received by the transmitting and receiving unit 31, or an acoustic ray corresponding to a boundary position on one of right and left sides of the region of interest set through the input unit 34.

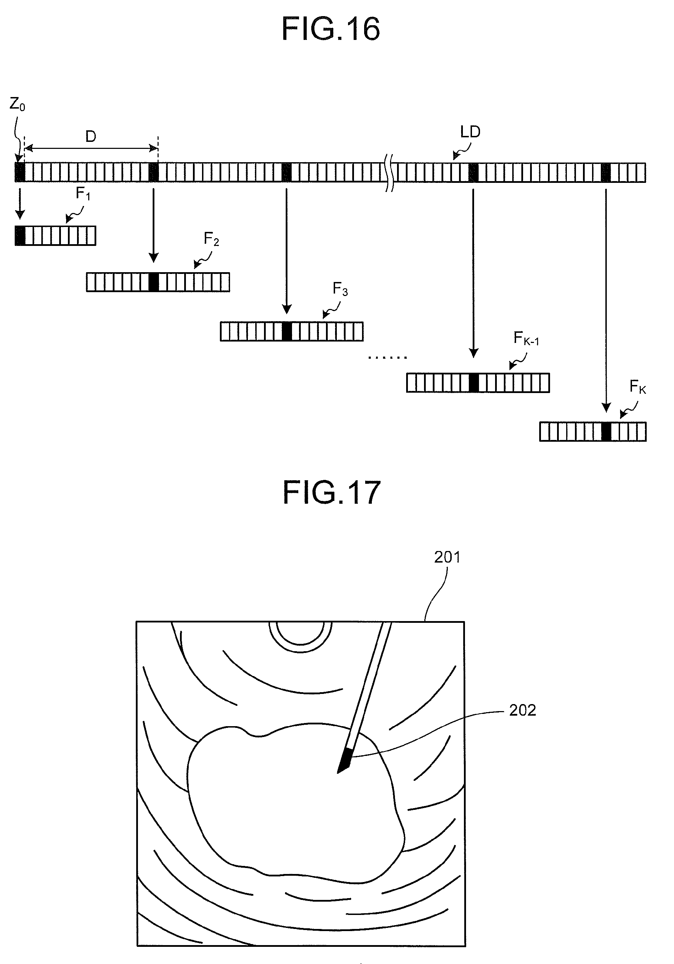

Next, the frequency analyzer 322 calculates frequency spectrums of all of a plurality of data positions set on one acoustic ray. First, the frequency analyzer 322 sets an initial value $Z_0$ of a data position Z (corresponding to the reception depth) representing a series of data groups (FFT data groups) acquired for FFT calculation (step S22). FIG. 16 is an exemplary view illustrating data arrangement of one acoustic ray. In an acoustic ray LD illustrated in FIG. 16, a white or black rectangle means one data. The acoustic ray LD is made discrete at time intervals corresponding to a sampling frequency (for example, 50 MHz) in A/D conversion performed by the transmitting and receiving unit 31. FIG. 16 illustrates a case where first data of the acoustic ray LD is set as the initial value $Z_0$ of the data position Z. FIG. 16 is a just an example, and the position of the initial value $Z_0$ can be arbitrarily set. For example, a data position Z corresponding to an upper end position of the region of interest may be set as the initial value $Z_0$.

Then, the frequency analyzer 322 acquires an FFT data group at the data position Z (step S23) and allows a window function stored in the window function storage unit 352 to act on the acquired FFT data group (step S24). When the window function acts on the FFT data group in this manner, discontinuity of the FFT data groups at the boundary can be avoided, and an artifact can be prevented from occurring.

Next, the frequency analyzer 322 determines whether or not the FFT data group at the data position Z is a normal data group (step S25). Here, it is necessary for the FFT data group to have the number of pieces of data being a power of two. Hereinafter, the FFT data group has $2^n$ (n is a positive integer) pieces of data. The normal FFT data group means that the data position Z is a $2^{n-1}$-th position from the front in the FFT data group. In other words, the normal FFT data group means that there are $2^{n-1}-1$ (= N) pieces of data before the data position Z, and there are $2^{n-1}$ (= M) pieces of data after the data position Z. In FIG. 16, n = 4 is set (N = 7, M = 8), and the FFT data groups $F_2$, $F_3$, and $F_{K-1}$ are normal, while the FFT data groups $F_1$ and $F_K$ are abnormal.

As a result of the determination in step S25, when the FFT data group at the data position Z is normal (Yes in step S25), the frequency analyzer 322 proceeds to step S27 to be described later.

As a result of the determination in step S25, when the FFT data group at the data position Z is not normal (No in step S25), the frequency analyzer 322 generates a normal FFT data group by inserting zero data by a shortfall (step S26). For the FFT data group which has been determined to be abnormal in step S25, a window function acts before the addition of zero data. Therefore, data discontinuity does not occur even when zero data is inserted into the FFT data group. After step S26, the frequency analyzer 322 proceeds to step S27 to be described later.

In Step S27, the frequency analyzer 322 obtains a frequency spectrum by performing FFT calculation using the FFT data group (step S27). As an example of such a frequency spectrum, there can be exemplified the spectrum curve $C_1$ illustrated in FIG. 6.

Next, the frequency analyzer 322 adds a specified data step width D to the data position Z to calculate a data position Z of a FFT data group which is a next analysis target (step S28). Here, it is desirable that the data step width D is made equal to a data step width used when the B-mode image data generator 331 generates the B-mode image data. However, when a calculation amount in the frequency analyzer 322 needs to be reduced, the data step width D may be set to be greater than the data step width which is used by the B-mode image data generator 331. FIG. 16 illustrates a case where D is 15.

Then, the frequency analyzer 322 determines whether or not the data position Z is greater than a final data position $Z_{max}$ (step S29). Here, the final data position $Z_{max}$ may be a data length of the acoustic ray LD, or may be a data position corresponding to a lower end of the region of interest. As a result of the determination, when the data position Z is greater than the final data position $Z_{max}$ (Yes in step S29), the frequency analyzer 322 increases an acoustic ray number L by 1 (step S30). On the other hand, when the data position Z is equal to or less than the final data position $Z_{max}$ (No in step S29), the frequency analyzer 322 returns to step S23. In this manner, regarding one acoustic ray LD, the frequency analyzer 322 performs FFT calculation on $[\{(Z_{max} - Z_0)/D\} + 1]$ (= K) FFT data groups. Here, [X] represents a maximum integer not exceeding X.

When the acoustic lay number L increased in step S30 is greater than a final acoustic ray number $L_{max}$ (Yes in step S31), the frequency analyzer 322 returns to the main routine illustrated in FIG. 14. On the other hand, when the acoustic lay number L increased in step S30 is equal to or less than the final acoustic ray number $L_{max}$ (No in step S31), the frequency analyzer 322 returns to step S22.

In this manner, the frequency analyzer 322 performs the FFT calculation K times for each of $(L_{max} - L_0 + 1)$ acoustic rays. The final acoustic ray number $L_{max}$ may be imparted to, for example, an acoustic ray which is finally received by the transmitting and receiving unit 31, or an acoustic ray corresponding to a boundary on one of the right and left sides of the region of interest.

Subsequent to the above-described frequency analysis processing in step S8, the attenuation corrector 323 performs attenuation correction on the frequency spectrum calculated by the frequency analyzer 322 using the FFT calculation (step S9). The attenuation corrector 323 calculates the data position z based on the sampling frequency of the data and assigns the data position z to the reception depth z of the above expression (1) to thereby calculate an attenuation amount A of the ultrasonic wave. Then, the attenuation corrector 323 uses the attenuation amount A calculated for each frequency to apply attenuation correction to the frequency spectrum. As a concrete example representing a result of the attenuation correction performed by the attenuation corrector 323, the spectrum curve $C_1'$ illustrated in FIG. 7 can be exemplified.

Here, a concrete example of the calculation that the attenuation corrector 323 calculates the data position Z will be described. When a sampling frequency of the data is 50 MHz, a time interval of data sampling is 1/50 (MHz) = 20 (nsec). Here, assuming that a speed of sound is 1,530 (m/sec), a distance interval of data sampling is 1,530 (m/sec) x 20 (nsec)/2 = 0.0153 (mm). Assuming that the data step width D from the first data of the acoustic ray LD to a data position of the FFT data group of the processing target is k, the data position Z is 0.0153k (mm).

Then, the feature data calculator 324 calculates feature data based on the frequency spectrum obtained in step S9 (step S10). Specifically, the feature data calculator 324 calculates, as the feature data, the bandwidth $\Delta f$ corresponding to the intensity width $\Delta I$ in which a maximum value of the frequency spectrum is set as an upper limit.

Thereafter, the artificial material detector 325 detects an artificial material using the bandwidth $\Delta f$ calculated by the feature data calculator 324 (step S11). Specifically, the artificial material detector 325 compares a magnitude between the bandwidth $\Delta f$ calculated by the feature data calculator 324 and threshold $\Delta f_{th}$ to detect a frequency spectrum in which $\Delta f < \Delta f_{th}$ is satisfied as a frequency spectrum of a reflection signal from the artificial material.

Then, the artificial material-highlighted image data generator 332 uses the B-mode image data generated by the B-mode image data generator 331 and a result of the detection from the artificial material detector 325 to generate an artificial material-highlighted image data (step S12).

Then, the controller 36 transmits the artificial material-highlighted image data generated by the artificial material-highlighted image data generator 332 to the display apparatus 5 (step S13). FIG. 17 is a view illustrating a display example of an artificial material-highlighted image displayed on the display apparatus 5. An artificial material-highlighted image 201 of FIG. 17 includes a highlighted portion 202 at which a position corresponding to the distal end of the puncture needle 101 is highlighted. A detected artificial material is thus highlighted in the artificial material-highlighted image 201, thereby allowing a user using the puncture needle 101 inside the body of the subject to grasp a position of the distal end of the puncture needle 101 easily and accurately.

The ultrasonic endoscope system 1 may periodically repeat the processing steps S1 to S13 described above.

According to the embodiment of the present invention described above, feature data reflecting a frequency spectrum shape specific to an artificial material having a specially regular structure is calculated, and then an image (artificial material-highlighted image) in which the artificial material is highlighted is generated based on the calculated feature data. With this configuration, it is possible to provide an ultrasonic observation apparatus capable of accurately detecting the position of an artificial material inserted into the body of the subject with a simple structure and having a display parameter capable of improving visibility of the artificial material in an ultrasonic image, an operation method therefor, and an operation program therefor.

Further, according to the embodiment, the artificial material-highlighted image data in which a detection result concerning the artificial material is highlighted is generated, whereby the visibility of the artificial material in the ultrasonic image can be improved.

Further, according to the embodiment, for detecting the artificial material, it is only necessary to perform signal processing in the apparatus and it is not necessary to use other external devices or a medical agent. This does not force a user to perform complicated operation.

(First Modification)

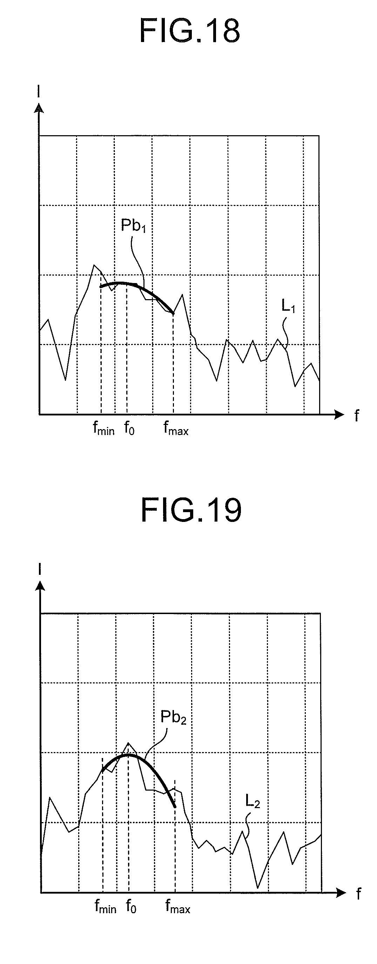

FIGS. 18 and 19 are views each explaining a brief overview of the feature data calculation processing and artificial material detection processing in a first modification of the present embodiment. In the first modification, the feature data calculator 324 approximates a specified frequency band $FB_1 = \{f | f_{min} \le f \le f_{max}\}$ with a quadratic (parabola) through frequency spectrum regression analysis and calculates a coefficient of a squared term in the approximate parabola as feature data. Here, the frequency band $FB_1$ is set so as to include, as an element, a peak frequency $P_0$ that imparts an intensity peak in the frequency spectrum of the distal end of the puncture needle 101. The peak frequency $f_0$ and frequency band FB are assumed to be previously stored in the feature data information storage unit 354.

The approximate parabola in the frequency band $FB_1$ is represented by the following expression (3):

$$I = p(f-f_a)^2 + q \cdots (3)$$

where $f_a \in F$ is satisfied. Further, the larger an absolute value $|p|$ of the coefficient p of the squared term, the more the parabola spreads. For example, when an approximate parabola $Pb_1$ of the living tissue illustrated in FIG. 18 and an approximate parabola $Pb_2$ of the distal end of the puncture needle 101 illustrated in FIG. 19 are compared, the approximate parabola $Pb_1$ has a larger $|p|$ value.

In the first modification, the artificial material detector 325 compares a threshold $|p_{th}|$ and absolute value $|p|$ of the coefficient p of the squared term and detects a frequency spectrum of an approximate parabola satisfying $|p| < |p_{th}|$ as a frequency spectrum from the artificial material.

(Second Modification)

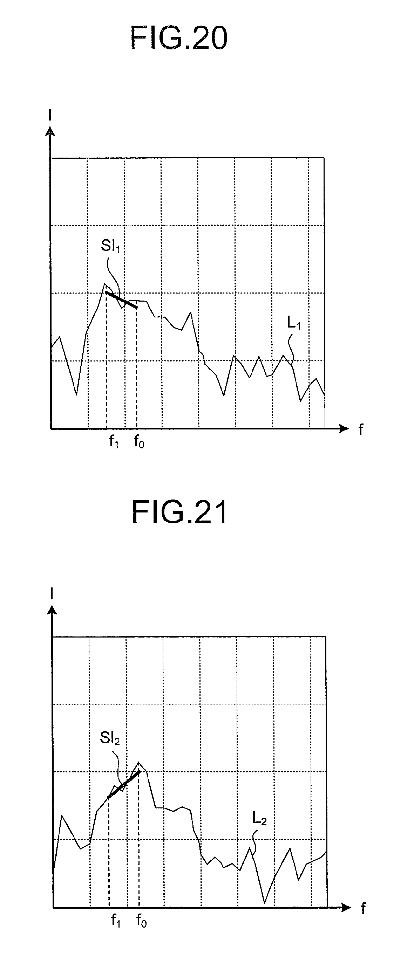

FIGS. 20 and 21 are views each explaining a brief overview of the feature data calculation processing and artificial material detection processing in a second modification of the present embodiment. In the second modification, the feature data calculator 324 approximates a frequency band $FB_2 = \{f | f_1 \le f \le f_0\}$ in which the peak frequency $f_0$ is set as the maximum value, by a straight line through regression analysis, and calculates a slope (coefficient of linear expression) of an approximate straight line as feature data. The peak frequency $f_0$ and frequency band $FB_2$ are assumed to be previously stored in the feature data information storage unit 354.

The approximate straight line in the frequency band $FB_2$ is represented by the following expression (4).

$$I = af + b \cdots (4)$$

Here, when an approximate straight line $Sl_1$ of the living tissue illustrated in FIG. 20 and an approximate straight line $Sl_2$ of the distal end of the puncture needle 101 illustrated in FIG. 21 are compared, the approximate straight line $Sl_2$ has a positive slope ($a>0$), while the approximate straight line $Sl_1$ has a negative slope ($a<0$).

In the second modification, the artificial material detector 325 compares a threshold $a_{th}$ ($>0$) and the slope a of the approximate straight line and detects a frequency spectrum of an approximate straight line satisfying $a > a_{th}$ as a frequency spectrum from the artificial material.

(Third Modification)

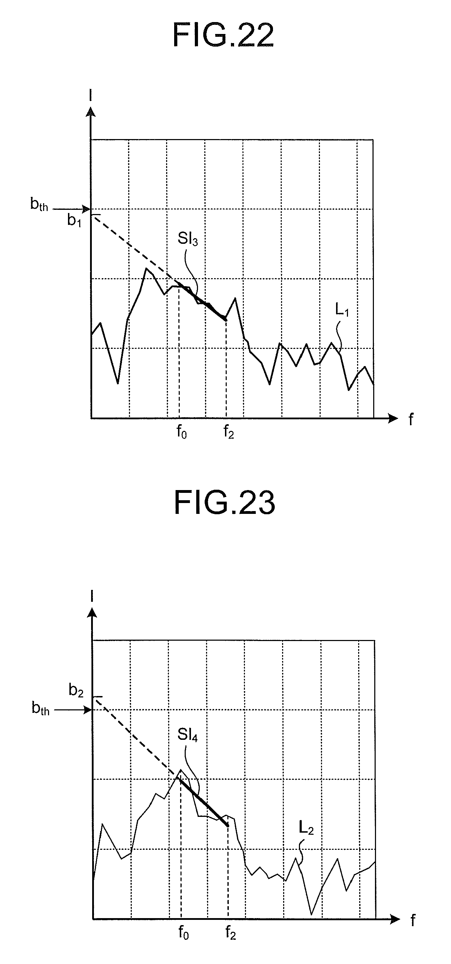

FIGS. 22 and 23 are views each explaining a brief overview of the feature data calculation processing and artificial material detection processing in a third modification of the present embodiment. In the third modification, the feature data calculator 324 approximates a frequency band $FB_3 = \{f | f_0 \le f \le f_2\}$ in which the peak frequency $f_0$ is set as the minimum value with a straight line through regression analysis (see expression (4)) and calculates an intercept b of an approximate straight line as feature data. The peak frequency $f_0$ and frequency band $FB_3$ are assumed to be previously stored in the feature data information storage unit 354.

When an approximate straight line $SI_3$ of the living tissue illustrated in FIG. 22 and an approximate straight line $SI_4$ of the distal end of the puncture needle 101 illustrated in FIG. 23 are compared, an absolute value of a negative slope from the peak is larger in the approximate straight line $SI_4$, so that an intercept of the approximate straight line $SI_4$ is larger than an intercept of the approximate straight line $SI_3$.

In the third modification, the artificial material detector 325 compares a threshold $b_{th}$ (>0) and the intercept b of the approximate straight line and detects a frequency spectrum of an approximate straight line satisfying $b > b_{th}$ as a frequency spectrum from the artificial material.

In the third modification, the frequency band for which the approximate straight line is calculated may be set to the same frequency band $FB_2$ used in the second modification. Further, in the third modification, the slope a may be used as the feature data.

(Fourth Modification)

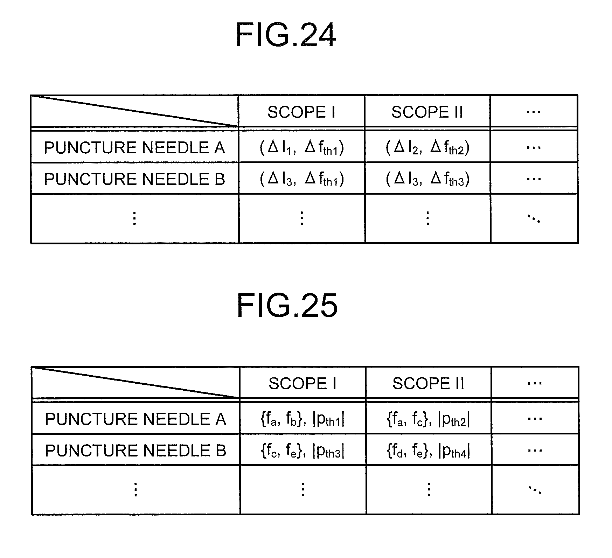

FIG. 24 is a view illustrating feature data information stored in the feature data information storage unit 354 in a fourth modification of the present embodiment. In the fourth embodiment, the feature data information storage unit 354 stores a combination of the intensity width and threshold for the band width in accordance with a combination of the ultrasonic endoscope 2 (scope) and the puncture needle 101. For example, a combination of the intensity width and threshold for the band width for detecting a puncture needle A inserted into a scope I is $(\Delta I_1, \Delta f_{th1})$. Further, a combination of the intensity width and threshold for the band width for detecting a puncture needle B inserted into a scope II is $(\Delta I_3, \Delta f_{th3})$.

In this manner, the feature data information storage unit 354 stores the feature data in accordance with a combination of types of the ultrasonic endoscope 2 and the puncture needle 101, thereby allowing the artificial material to be detected with higher accuracy in accordance with the combination of types of the ultrasonic endoscope 2 and the puncture needle 101.

FIG. 25 is a view illustrating another example of the feature data information stored in the feature data information storage unit 354 in the fourth modification of the present embodiment. In the example of FIG. 25, the feature data information storage unit 354 sets the frequency band and a threshold for spread of the parabola (coefficient of a squared term) in accordance with in accordance with a combination of the ultrasonic endoscope 2 (scope) and the puncture needle 101. For example, a frequency band and a threshold for the coefficient of a squared term in the approximate parabola for detecting a puncture needle A inserted into a scope I is $\{f_a, f_b\}$ and $|p_{th1}|$, respectively. Further, a frequency band and a threshold for spread of the parabola for detecting a puncture needle B inserted into the scope I is $\{f_c, f_e\}$ and $|p_{th3}|$, respectively. Here, {A, B} means that the frequency band satisfies $\{f | A \le f \le B\}$.

The feature data information storage unit 354 may store the frequency band and a threshold for the slope or intercept of the approximate parabola as the feature data information.

Further, the feature data information storage unit 354 may store all the combinations described in the fourth modification 4 as the feature data information. In this case, a configuration may be employed in which a user can select arbitrary one of the feature data for detection of the artificial material through the input unit 34 or in which the artificial material detector 325 performs processing for all the feature data and detects the artificial material based on results of the processing. In the latter case, the artificial material detector 325 may detect, as the artificial material, an object that has been determined by a specified number of the plurality of feature data.

Since the extending angle of the puncture needle 101 is constant depending on the type of the ultrasonic endoscope 2, the extending angle itself may be stored as the feature data information. In this case, a neighboring region in the extending direction of the puncture needle 101 may be defined as a target region of the feature data calculation to be performed by the feature data calculator 324 or as a target region of the artificial material-highlighted image data generation to be performed by the artificial material-highlighted image data generator 332.

(Other Embodiments)

Although the embodiment of the invention has been described as above, the invention is not limited only to the above-described embodiment. For example, in the present invention, the artificial material may be detected using a peak value of the intensity of the frequency spectrum.

Further, in the present invention, the concave portion is not formed at the distal end of the puncture needle, the artificial material-highlighted image data may be generated including a distance between a concave portion closest to a distal end position and the distal end position.

Further, in the present invention, the ultrasonic observation system may be of a type that performs observation by bringing the ultrasonic probe into contact with the body surface of the subject. As an artificial material to be detected by the ultrasonic observation apparatus, there can be exemplified a puncture needle to be inserted from the body surface of the subject into inside thereof.

The present invention may be applied as highlighting technology for a full range of objects having a spatially regular structure. Thus, even the living tissue can be highlighted, if it has a spatially regular structure.

As described above, the present invention can be implemented in various forms without departing from the technical idea described in the claims.

Reference Signs List

| 1 | ultrasonic endoscope system |
|---|---|
| 2 | ultrasonic endoscope |
| 3 | ultrasonic observation apparatus |
| 4 | endoscope observation apparatus |
| 5 | display apparatus |
| 6 | light source apparatus |
| 7 | ultrasonic cable |
| 8 | video cable |
| 9 | optical fiber cable |
| 21 | insertion unit |
| 22 | operation unit |
| 23 | universal cable |
| 24 | connector |
| 31 | transmitting and receiving unit |
| 32 | calculator |
| 33 | image processor |
| 34 | input unit |
| 35 | storage unit |
| 36 | controller |
| 101 | puncture needle |
| 102 | concave portion |
| 201 | artificial material-highlighted image |
| 202 | highlighted portion |
| 211 | ultrasonic probe |
| 212 | hard portion |
| 212a | treatment tool channel |
| 213 | bending portion |
| 214 | flexible tube portion |
| 215 | transducer unit |
| 221 | bending knob |
| 222 | operation member |
| 223 | treatment tool insertion port |
| 311 | echo signal amplifier |
| 321 | amplification corrector |
| 322 | frequency analyzer |
| 323 | attenuation corrector |
| 324 | feature data calculator |
| 325 | artificial material detector |
| 331 | B-mode image data generator |
| 332 | artificial material-highlighted image data generator |
| 351 | amplification factor information storage unit |
| 352 | window function storage unit |
| 353 | correction information storage unit |
| 354 | feature data information storage unit |

## Claims

1.  An ultrasonic observation apparatus that receives an echo signal of an ultrasonic wave from an ultrasonic probe for transmitting an ultrasonic wave to a subject and receiving the ultrasonic wave reflected from the subject and performs signal processing on the echo signal, the ultrasonic observation apparatus comprising:

a frequency analyzer that analyzes a frequency of the echo signal to calculate a frequency spectrum;
a feature data calculator that calculates feature data reflecting a frequency spectrum shape specific to an artificial material having a spatially regular structure; and
an artificial material-highlighted image data generator that generates, based on the feature data, an image in which the artificial material is highlighted.

2. The ultrasonic observation apparatus according to claim 1, further comprising an artificial material detector that detects, based on the feature data calculated by the feature data calculator, information on the artificial material, wherein
the artificial material-highlighted image data generator generates, based on the feature data and a detection result by the artificial material detector, the image in which the artificial material is highlighted.

3. The ultrasonic observation apparatus according to claim 1, wherein the artificial material is a puncture needle on a surface of a distal end of which a plurality of concave portions are arranged to form a spatially regular pattern.

4. The ultrasonic observation apparatus according to claim 3, wherein
the ultrasonic probe can receive an ultrasonic wave satisfying
$2d\sin\theta = n\lambda$ (n is an integer)
where an interval between the concave portions adjacently disposed is d, a wavelength of the ultrasonic wave is $\lambda$, and, an incident angle of the ultrasonic wave incident on a surface of the puncture needle is $\theta$.

5. The ultrasonic observation apparatus according to claim 1, wherein
the feature data calculator calculates, as the feature data, a frequency band that imparts an intensity within a specified range from a maximum value of the intensity of the frequency spectrum.

6. The ultrasonic observation apparatus according to claim 1, wherein
the feature data calculator approximates a frequency band in the frequency spectrum that includes a frequency characterizing the artificial material, by a quadratic curve through regression analysis, and calculates, as the feature data, a coefficient of a squared term in the quadratic curve.

7. The ultrasonic observation apparatus according to claim 1, wherein
the feature data calculator approximates a frequency band in the frequency spectrum that includes a frequency characterizing the artificial material, by a straight line through regression analysis, and calculates, as the feature data, a slope of the straight line.

8. The ultrasonic observation apparatus according to claim 1, wherein
the feature data calculator approximates a frequency band in the frequency spectrum that includes a frequency characterizing the artificial material, by a straight line through regression analysis, and calculates, as the feature data, an intercept of the straight line.

9. The ultrasonic observation apparatus according to claim 1, further comprising a feature data information storage unit that stores feature data information which is information required for the feature data calculator to calculate the feature data, wherein
the feature data calculator calculates, based on information stored in the feature data information storage unit, the feature data.

10. An operation method of an ultrasonic observation apparatus that receives an echo signal from an ultrasonic probe for transmitting an ultrasonic wave to a subject and receiving the ultrasonic wave reflected from the subject and performs signal processing on the echo signal, the operation method comprising:

analyzing, by a frequency analyzer, a frequency of the echo signal to calculate a frequency spectrum;
calculating, by a feature data calculator, feature data reflecting a frequency spectrum shape specific to an artificial material having a spatially regular structure; and
generating, by an artificial material-highlighted image data generator, an image in which the artificial material is highlighted, based on the feature data.

**11.** An operating program for an ultrasonic observation apparatus that receives an echo signal from an ultrasonic probe for transmitting an ultrasonic wave to a subject and receiving the ultrasonic wave reflected from the subject and performs signal processing on the echo signal, the program causing the ultrasonic observation apparatus to perform:

analyzing, by a frequency analyzer, a frequency of the echo signal to calculate a frequency spectrum;
calculating, by a feature data calculator, feature data reflecting a frequency spectrum shape specific to an artificial material having a spatially regular structure; and
generating, by an artificial material-highlighted image data generator, an image in which the artificial material is highlighted, based on the feature data.

# FIG.1

ENDOSCOPE
OBSERVATION
APPARATUS
4

LIGHT
SOURCE
APPARATUS
6

ULTRASONIC
OBSERVATION
APPARATUS
3

# FIG.2

211    212

215

212a

101

# FIG.3

ULTRASONIC OBSERVATION APPARATUS (3)

INPUT UNIT (34)

TRANSMITTING AND RECEIVING UNIT (31)
- ECHO SIGNAL AMPLIFIER (311)

CONTROLLER (36)

STORAGE UNIT (35)
- AMPLIFICATION FACTOR INFORMATION STORAGE UNIT (351)
- WINDOW FUNCTION STORAGE UNIT (352)
- CORRECTION INFORMATION STORAGE UNIT (353)
- FEATURE DATA INFORMATION STORAGE UNIT (354)

CALCULATOR (32)
- AMPLIFICATION CORRECTOR (321)
- FREQUENCY ANALYZER (322)
- ATTENUATION CORRECTOR (323)
- FEATURE DATA CALCULATOR (324)
- ARTIFICIAL MATERIAL DETECTOR (325)

IMAGE PROCESSOR (33)
- B-MODE IMAGE DATA GENERATOR (331)
- ARTIFICIAL MATERIAL-HIGHLIGHTED IMAGE DATA GENERATOR (332)

EP 2 878 271 A1

# FIG.4

# FIG.5

# FIG.6

# FIG.7

$$A = 2\,\alpha\,zf_1$$

# FIG.8

102

101

# FIG.9

d

θ

102

dsin θ

102

# FIG.10

# FIG.11

# FIG.12

# FIG.13

# FIG.14

```
                    ┌──────────┐
                    │  START   │
                    └──────────┘
                         │
                         ▼
          ┌─────────────────────────────┐
          │   MEASURE NEW SUBJECT        │──S1
          └─────────────────────────────┘
                         │
                         ▼
          ┌─────────────────────────────┐
          │ AMPLIFY RECEIVED ECHO SIGNAL │──S2
          │      (STC CORRECTION)        │
          └─────────────────────────────┘
                         │
                         ▼
          ┌─────────────────────────────┐
          │ GENERATE B-MODE IMAGE DATA   │──S3
          └─────────────────────────────┘
                         │
                         ▼
          ┌─────────────────────────────┐
          │    DISPLAY B-MODE IMAGE      │──S4
          └─────────────────────────────┘
                         │
                         ▼
                      ╱     ╲   S5
                   ╱  HAS REGION  ╲       NO
                 ╱  OF INTEREST BEEN ╲──────────────┐
                 ╲      SET?        ╱                │
                   ╲             ╱                   ▼
                      ╲       ╱                   ╱     ╲   S7
                         │YES                   ╱  END?  ╲──── NO
                         ▼                      ╲       ╱
          ┌─────────────────────────────┐          │YES
          │   AMPLIFICATION CORRECTION   │──S6       │
          └─────────────────────────────┘           │
                         │                           │
                         ▼                           │
          ┌─────────────────────────────┐            │
          │     FREQUENCY ANALYSIS       │──S8        │
          └─────────────────────────────┘            │
                         │                            │
                         ▼                            │
          ┌─────────────────────────────┐             │
          │ PERFORM ATTENUATION          │──S9         │
          │ CORRECTION ON FREQUENCY      │             │
          │ SPECTRUM                     │             │
          └─────────────────────────────┘             │
                         │                             │
                         ▼                             │
          ┌─────────────────────────────┐              │
          │   CALCULATE FEATURE DATA     │──S10         │
          └─────────────────────────────┘              │
                         │                              │
                         ▼                              │
          ┌─────────────────────────────┐               │
          │  DETECT ARTIFICIAL MATERIAL  │──S11          │
          └─────────────────────────────┘               │
                         │                               │
                         ▼                               │
          ┌─────────────────────────────┐                │
          │ GENERATE ARTIFICIAL MATERIAL-│──S12           │
          │  HIGHLIGHTED IMAGE DATA      │                │
          └─────────────────────────────┘                │
                         │                                │
                         ▼                                │
          ┌─────────────────────────────┐                 │
          │ TRANSMIT ARTIFICIAL MATERIAL-│──S13            │
          │  HIGHLIGHTED IMAGE DATA      │                 │
          └─────────────────────────────┘                 │
                         │◄───────────────────────────────┘
                         ▼
                    ┌──────────┐
                    │   END    │
                    └──────────┘
```

# FIG.15

```
┌──────────────────┐
│    FREQUENCY     │
│    ANALYSIS      │
└──────────────────┘
         │
         ▼
┌──────────────────────────┐
│         $L = L_0$         │ ～ S21
└──────────────────────────┘
         │
         ▼
┌──────────────────────────┐
│         $Z = Z_0$         │ ～ S22
└──────────────────────────┘
         │
         ▼
┌──────────────────────────┐
│   ACQUIRE FFT DATA GROUP  │ ～ S23
└──────────────────────────┘
         │
         ▼
┌──────────────────────────┐
│    APPLY WINDOW FUNCTION  │ ～ S24
└──────────────────────────┘
         │
         ▼
      ╱────────╲  ～ S25
     ╱    IS    ╲         NO
    ╱ NORMAL FFT ╲──────────────┐
    ╲   DATA     ╱              │
     ╲  GROUP?  ╱               ▼
      ╲────────╱      ┌──────────────────┐ ～ S26
         │ YES        │  INSERT ZERO DATA │
         │            │   BY SHORTFALL    │
         │            └──────────────────┘
         │                     │
         ◄─────────────────────┘
         │
         ▼
┌──────────────────────────┐
│     FFT CALCULATION       │ ～ S27
└──────────────────────────┘
         │
         ▼
┌──────────────────────────┐
│        $Z = Z + D$        │ ～ S28
└──────────────────────────┘
         │
         ▼
      ╱────────╲  ～ S29
     ╱$Z > Z_{max}$?╲   NO
     ╲          ╱──────────────┐
      ╲────────╱               │
         │ YES                 │
         ▼                     │
┌──────────────────────────┐   │
│        $L = L + 1$        │ ～ S30
└──────────────────────────┘   │
         │                     │
         ▼                     │
  NO  ╱────────╲  ～ S31        │
 ┌────╱$L > L_{max}$?╲          │
 │    ╲          ╱              │
 │     ╲────────╱               │
 │        │ YES                 │
 │        ▼                     │
 │  ┌──────────┐                │
 │  │  RETURN  │                │
 │  └──────────┘                │
 └──────────────────────────────
```

# FIG.16

# FIG.17

# FIG.18

# FIG.19

# FIG.20

# FIG.21

# FIG.22

# FIG.23

# FIG.24

| | SCOPE I | SCOPE II | ··· |
|---|---|---|---|
| PUNCTURE NEEDLE A | $(\Delta I_1, \Delta f_{th1})$ | $(\Delta I_2, \Delta f_{th2})$ | ··· |
| PUNCTURE NEEDLE B | $(\Delta I_3, \Delta f_{th1})$ | $(\Delta I_3, \Delta f_{th3})$ | ··· |
| ⋮ | ⋮ | ⋮ | ⋱ |

# FIG.25

| | SCOPE I | SCOPE II | ··· |
|---|---|---|---|
| PUNCTURE NEEDLE A | $\{f_a, f_b\}, |p_{th1}|$ | $\{f_a, f_c\}, |p_{th2}|$ | ··· |
| PUNCTURE NEEDLE B | $\{f_c, f_e\}, |p_{th3}|$ | $\{f_d, f_e\}, |p_{th4}|$ | ··· |
| ⋮ | ⋮ | ⋮ | ⋱ |

**INTERNATIONAL SEARCH REPORT**

| International application No. |
|---|
| PCT/JP2013/067911 |

A. CLASSIFICATION OF SUBJECT MATTER
*A61B8/12*(2006.01)i

According to International Patent Classification (IPC) or to both national classification and IPC

B. FIELDS SEARCHED

Minimum documentation searched (classification system followed by classification symbols)
A61B8/12

Documentation searched other than minimum documentation to the extent that such documents are included in the fields searched
Jitsuyo Shinan Koho       1922-1996   Jitsuyo Shinan Toroku Koho   1996-2013
Kokai Jitsuyo Shinan Koho  1971-2013   Toroku Jitsuyo Shinan Koho   1994-2013

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2009-254780 A  (Fujifilm Corp.), 05 November 2009 (05.11.2009), entire text; all drawings & US 2009/0247873 A1 | 1-11 |
| A | JP 10-057376 A  (GE Yokogawa Medical Systems, Ltd.), 03 March 1998 (03.03.1998), entire text; all drawings (Family: none) | 1-11 |
| A | JP 2010-194013 A  (Hoya Corp.), 09 September 2010 (09.09.2010), entire text; all drawings (Family: none) | 1-11 |

[X] Further documents are listed in the continuation of Box C.    [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search 16 July, 2013 (16.07.13) | Date of mailing of the international search report 30 July, 2013 (30.07.13) |
|---|---|
| Name and mailing address of the ISA/ Japanese Patent Office | Authorized officer |
| Facsimile No. | Telephone No. |

Form PCT/ISA/210 (second sheet) (July 2009)

31

**INTERNATIONAL SEARCH REPORT**

International application No.

PCT/JP2013/067911

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2004-181095 A  (Olympus Corp.),<br>02 July 2004 (02.07.2004),<br>entire text; all drawings<br>& US 2004/0249288 A1    & EP 1426011 A1 | 1-11 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006175006 A
- JP 2005323669 A